# EUROPÄISCHE PATENTSCHRIFT

(11) **EP 0 990 421 B1**
(45) Veröffentlichungstag und Bekanntmachung des Hinweises auf die Patenterteilung: **16.03.2005**
(21) Anmeldenummer: 99810790.8
(22) Anmeldetag: 02.09.1999
(51) Int. Cl.: A61B 17/16

(54) **Hohlfräser für Gewebe**
Hollow mill for tissue
Fraise creuse pour tissus

(30) Priorität: 29.09.1998 EP 98810978
(43) Veröffentlichungstag der Anmeldung: 05.04.2000
(73) Patentinhaber: Zimmer GmbH, 8404 Winterthur (CH)
(72) Erfinder: Zech, Manfred, 8450 Andelfingen (CH); Favre, Alain, 8052 Zürich (CH)
(74) Vertreter: Manitz, Finsterwald & Partner Gbr

(56) Entgegenhaltungen:
- EP-A- 0 574 736
- EP-A- 0 611 624
- EP-A- 0 824 893
- DE-A- 3 408 313
- GB-A- 2 040 741
- JP-A- 60 090 547
- US-A- 4 538 944
- US-A- 5 632 747

## Beschreibung

Die Erfindung betrifft einen Hohlfräser gemäss dem Oberbegriff des unabhängigen Patentanspruchs.

Ein derartiger Hohlfräser ist aus US 5,632,747 bekannt.

Derartige Hohlfräser werden beispielsweise bei der Transplantation von Gewebesäulen eingesetzt. Beispielsweise werden Knorpeldefekte an belasteten Stellen wie z.B. Gelenken heutzutage in einigen Fällen derart "repariert", dass an der Defektstelle eine Sacklochbohrung erstellt wird, die bis in den subchondralen Knochen hinein reicht. An einer wenig belasteten Erntestelle wird dann eine Gewebesäule entnommen, welche aus Knochen besteht, die mit gesundem Knorpel bedeckt ist. Diese Gewebesäule wird zur Defektstelle transportiert und dort in das gebohrte Sackloch hinein implantiert.

Zur Erstellung der Gewebesäule an der Erntestelle sind verschiedene Instrumente vorgeschlagen worden wie z.B. Stanzen oder Hohlfräser (teilweise auch als "Kronenbohrer" bezeichnet). Ein solcher Hohlbohrer weist stirnseitig angeordnete Zähne auf, welche bei der Erstellung der Gewebesäule das Gewebe abtragen. Das abgetragene Gewebe kann durch den Innenraum des Hohlfräsers oder über den Raum, der den Hohlfräser umgibt, abtransportiert werden.

Beim Erstellen der Gewebesäule wird Knochengewebe abgetragen. Dabei erwärmt sich das Gewebe in unmittelbarer Nähe des distalen Endes des Fräsers (spanabhebendes Verfahren). Andererseits enthält das Knochengewebe Stoffe wie z.B. Eiweisse, welche nur eine sehr begrenzte Temperaturerhöhung vertragen, weil sie sonst denaturieren können. Dabei kann sowohl unmittelbar aussen um den Fräser herum angeordnetes Gewebe von einer solchen Temperaturerhöhung betroffen sein als auch unmittelbar von dem Fräser umgebenes Gewebe, also die Gewebesäule oder zumindest Teile davon.

Ein zahnmedizinisches Rotationswerkzeug mit Einrichtungen zur Kühlung einer Arbeitsfläche des Werkzeugs und der Zahnsubstanz ist in DE 3 408 313 A1 beschrieben.

Es ist eine Aufgabe der Erfindung einen Hohlfräser vorzuschlagen, bei welchem sichergestellt ist, dass weder das an der Erntestelle den Fräser unmittelbar umgebende Gewebe noch dasjenige der Gewebesäule bei der Erstellung und der Entnahme der Gewebesäule beschädigt werden kann.

Diese Aufgabe wird mit Hilfe eines Hohlfräsers gelöst, wie er durch die Merkmale des unabhängigen Patentanspruchs gekennzeichnet ist. Vorteilhafte Ausgestaltungen des erfindungsgemässen Hohlfräsers ergeben sich aus den Merkmalen der abhängigen Patentansprüche.

Der erfindungsgemässe Hohlfräser weist also insbesondere Mittel zum Kühlen des nahe dem distalen Ende gelegenen Bereichs des Fräsers beim Abtragen von Gewebe aufweist. Dadurch wird vermieden, dass der Fräser in dem Bereich, mit welchem er mit dem Gewebe in Kontakt kommt, eine Temperatur erreichen kann, die für das Gewebe schädlich sein kann.

Erfindungsgemäß umfassen die Mittel zum Kühlen mindestens eine in der Aussenwand des Fräsers spiralförmig verlaufende Nut, deren Orientierung entgegengesetzt zur Drehrichtung beim Abtragen von Gewebe ist, sodass beim Abtragen von Gewebe ein Kühlmittel, z.B. eine physiologische Salzlösung, durch die Nut zum distalen Ende des Fräsers gelangen kann. Es wird also ein (körperverträgliches) Kühlmittel aktiv zugeführt. Durch die Tatsache, dass die Orientierung der spiralförmigen Nut entgegengesetzt der Drehrichtung beim Abtragen von Gewebe ist, kann das zugeführte Kühlmittel auch an den Ort gelangen, wo die Kühlung erfolgen soll, nämlich zu dem Bereich nahe dem distalen Ende des Fräsers, wo der Fräser mit dem Gewebe in Kontakt kommt. Das zugeführte Kühlmittel kann zusammen mit dem abgetragenen Gewebe durch den Innenraum des Hohlfräsers abtransportiert werden.

Bei einem Ausführungsbeispiel können die Mittel zum Kühlen diskrete Durchgangsbohrungen umfassen, die nahe dem distalen Ende in der Wand des Fräsers ausgebildet sind. Dies ist eine besonders einfache konstruktive Massnahme.

Hierbei können in Umfangsrichtung betrachtet mehrere Durchgangsbohrungen gleichmässig zueinander beabstandet in der Wand des Fräsers angeordnet sind. In axialer Richtung betrachtet können mehrere Lagen solcher in Umfangsrichtung gleichmässig zueinander beabstandeter Durchgangsbohrungen in der Wand des Fräsers vorgesehen sein.

Bei einem weiteren Ausführungsbeispiel des erfindungsgemässen Hohlfräsers spitzen sich die stirnseitig angeordneten Schneidezähne von der Innenwand beginnend in Richtung auf das distale Ende des Hohlfräsers hin konisch nach aussen zu oder umgekehrt (d.h. sie spitzen sich von aussen nach innen zu). Dadurch wird erreicht, dass der Fräser auch dann nicht abgleiten kann, wenn er einmal nicht ganz genau orthogonal zur Schnittfläche aufgesetzt wird.

Die Spitzen der stirnseitig angeordneten Schneidezähne können (vorzugsweise mit einem sehr kleinen Radius) abgerundet sein, wodurch die Standzeit der Schneidezähne erhöht wird.

Im Bereich des distalen Endes des Fräsers kann der Innendurchmesser in proximaler Richtung betrachtet zunächst konstant sein und sich dann vergrössern. Auch kann im Bereich des distalen Endes des Fräsers der Aussendurchmesser in proximaler Richtung betrachtet zunächst konstant sein und sich dann verringern. Nachdem die Gewebesäule erstellt worden ist, muss der Hohlfräser wieder herausgenommen werden, während die Gewebesäule noch an ihrem Boden an der Erntestelle verankert ist. Sie wird anschliessend mit einem geeigneten Extraktor an ihrem Boden abgetrennt und kann dann entnommen und zur Defektstelle transportiert werden. Die beiden beschriebenen Massnahmen dienen nun dazu, dieses Herausnehmen des Hohlfräsers zu erleichtern, sie können auch beide kombiniert vorhanden sein. Ist der Innendurchmesser im Bereich des distalen Endes in proximaler Richtung betrachtet zunächst konstant und vergrössert sich sodann, heisst dies, dass die Gewebesäule nur an ihrem distalen Ende noch in Kontakt mit der Innenwand des Hohlfräsers steht. Der Hohlfräser kann dadurch leichter herausgenommen werden, als wenn die Gewebesäule über ihre gesamte Länge in Kontakt mit der Innenwand des Hohlfräsers stünde. Was den Kontakt mit dem umliegenden Gewebe betrifft, gilt das Gleiche. Der Hohlfräser ist nur am distalen Ende noch in Kontakt mit dem umliegenden Gewebe, weil der Aussendurchmesser des Hohlfräsers in proximaler Richtung betrachtet sich dann verringert. Dies erleichtert ebenfalls das Herausnehmen des Hohlfräsers. Dabei ist zu beachten, dass es sich hier nur um geringe Änderungen der Durchmesser handelt, weil die ohnehin nicht beliebig grosse Wandstärke des Hohlfräsers nicht beliebig reduziert werden kann, immerhin müssen beim Fräsen des subchondralen Knochens doch recht erhebliche Kräfte auf die Schnittfläche übertragen werden.

Im Bereich des distalen Endes des Hohlfräsers können auf der Aussenwand Markierungen vorgesehen sein, die dem Chirurgen bzw. Orthopäden die jeweils aktuelle Eindringtiefe des Hohlfräsers anzeigen und es ihm dadurch erleichtern, eine Gewebesäule einer genau bestimmten Länge zu erstellen.

Ferner können auf der Aussenwand des Fräsers Markierungen vorgesehen sind, welche sonstige Informationen über den Fräser enthalten (z.B. Fräserinnendurchmesser, Fräseraussendurchmesser, etc.). Diese sind vorzugsweise allerdings in einem anderen Bereich angebracht als diejenigen Markierungen, welche die aktuelle Eindringtiefe des Hohlfräsers anzeigen.

Weitere vorteilhafte Ausgestaltungen ergeben sich aus der Beschreibung des erfindungsgemässen Hohlfräsers anhand der Zeichnung. Es zeigen, teilweise schematisch und/oder im Schnitt:
- Fig. 1: ein Beispiel für einen nicht under den unabhängigen Patentanspruch fallenden, jedoch für das Verständnis der Erfindung dienlichen Hohlfräser in perspektivischer Ansicht,
- Fig. 2: den Hohlfräser aus Fig. 1 im Längsschnitt,
- Fig. 3: den Bereich des distalen Endes eines Ausführungsbeispiels des erfindungsgemässen Hohlfräsers
und
- Fig. 4: ein Ausführungsbeispiel für die Ausgestaltung der stirnseitigen Zähne ("Bohrkrone") eines erfindungsgemässen Hohlfräsers.

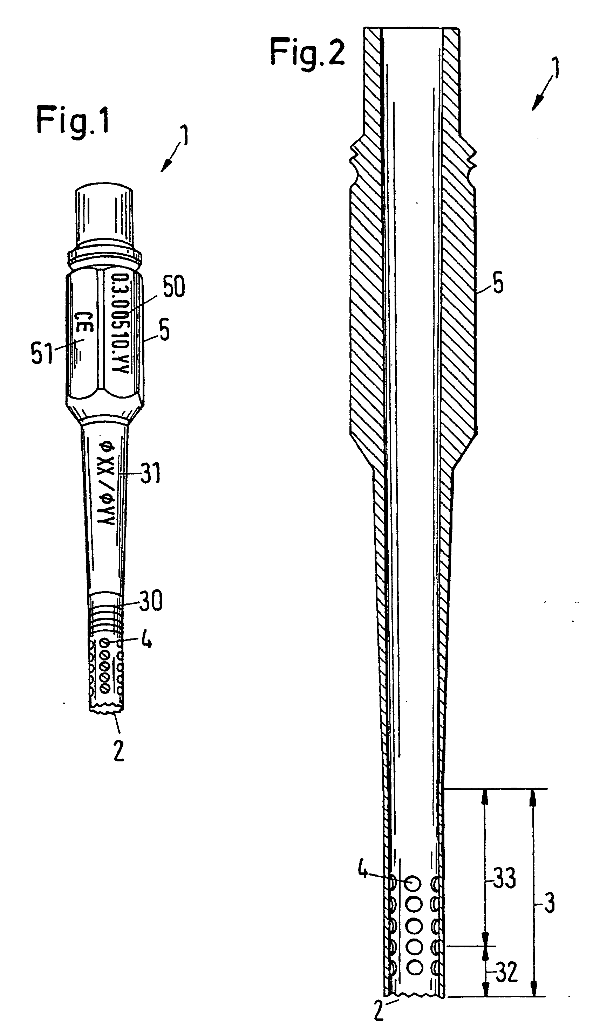

Das Beispiel des Hohlfräsers 1 gemäss Fig. 1, welches in Fig. 2 im Längsschnitt (vergrössert) dargestellt ist, lässt erkennen, dass der Hohlfräser 1 am distalen Ende stirnseitig angeordnete Zähne 2 aufweist. In einem Bereich 3 nahe dem distalen Ende ist der Hohlfräser 1 in seiner Wand mit diskreten Durchgangsbohrungen 4 versehen. Dabei sind in Umfangsrichtung betrachtet mehrere Durchgangsbohrungen 4 vorgesehen, und in axialer Richtung betrachtet sind mehrere Lagen solcher in Umfangsrichtung gleichmässig zueianander beabstandeter Durchgangsbohrungen 4 vorgesehen. Diese Durchgangsbohrungen bewirken eine Kühlung des Hohlfräsers 1 im Bereich 3 nahe dem distalen Ende, denn dort kommt der Hohlfräser mit dem Gewebe in Kontakt.

Im proximalen Bereich weist der Hohlfräser einen als Sechskant ausgebildeten Bereich 5 auf, welcher in einem Futter (nicht dargestellt) eines konventionellen Drehantriebs (chirurgische Bohrmaschine) aufgenommen und eingespannt werden kann, sodass der Hohlfräser von diesem Drehantrieb rotatorisch angetrieben werden kann.

Wie man in Fig. 1 erkennt, können auf der Aussenwand des Hohlfräsers verschiedene Markierungen (z.B. durch Laserbeschriftung) aufgebracht sein. Im Bereich 3 können dies Markierungen 30 sein, welche dem Chirurgen bzw. Orthopäden die Eindringtiefe des Hohlfräsers 1 anzeigen. Diese Markierungen 30 können bis zum distalen Ende hin auf der Aussenwand vorgesehen sein, also insbesondere auch in den Bereichen zwischen in Umfangsrichtung benachbarten Durchgangsbohrungen 4. Aus Gründen der besseren Übersichtlichkeit ist hierauf jedoch in Fig. 1 verzichtet worden. Weiter proximal können z.B. Markierungen 31 vorgesehen sein, welche Informationen über den Aussendurchmesser und den Innendurchmesser im Bereich des distalen Endes des Hohlfräsers 1 geben (codiert oder unkodiert). In dem Bereich 5, der als Sechskant ausgebildet ist, können dies z.B. herstellerspezifische Informationen 50 sein (Herstellungsseriennummern, Typennummern, etc.) oder auch Zertifizierungszeichen 51.

Weiterhin kann der Innendurchmesser in dem Bereich 3 nahe dem distalen Ende des Hohlfräsers 1 zunächst konstant sein (Bereich 32) und sich dann leicht vergrössern (Bereich 33). Dies ist in Fig. 2 praktisch nicht zu erkennen, weil die Vergrösserung des Innendurchmessers auch nicht beliebig sein kann, da die Wandstärke des Hohlfräsers nicht beliebig klein sein kann, immerhin müssen die für das Fräsen erforderlichen Kräfte auf die Schnittfläche übertragen werden. Zweck der Vergrösserung des Innendurchmessers im Bereich 33 ist, dass die Gewebesäule nur in dem Bereich 32 noch Kontakt mit der Innenwand des Hohlfräsers 1 aufweist, sodass der Hohlfräser nach Beendigung des Fräsvorgangs leichter herausgenommen werden kann.

Zu dem gleichen Zweck kann auch der Aussendurchmesser in dem Bereich 3 nahe dem distalen Ende des Hohlfräsers 1 zunächst konstant sein (Bereich 32) und sich dann leicht verringern. Auch dies ist in Fig. 2 praktisch nicht zu erkennen aus den bereits vorstehend genannten Gründen. Beide Massnahmen können kombiniert vorhanden sein oder nur eine der Massnahmen.

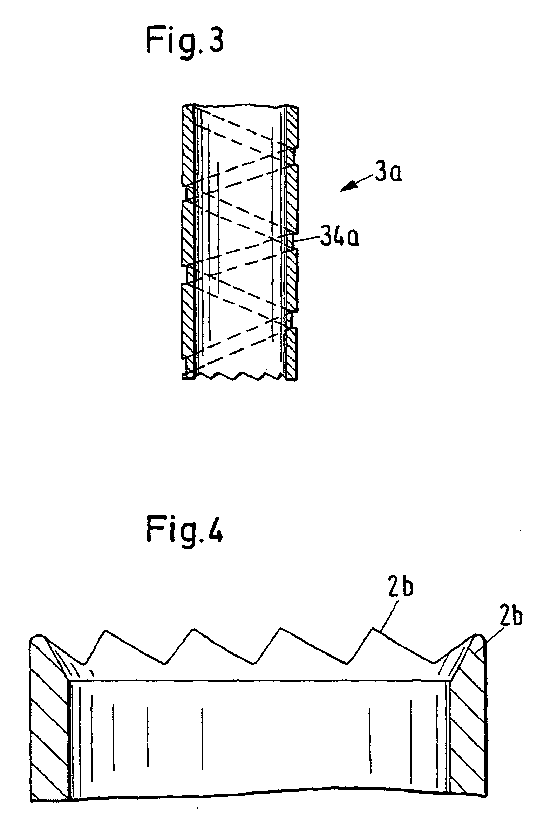

In Fig. 3 ist der Bereich 3a nahe dem distalen Ende eines Ausführungsbeispiels des erfindungsgemässen Hohlfräsers dargestellt. Man erkennt hier, dass die Mittel zum Kühlen eine in der Aussenwand des Hohlfräsers spiralförmig umlaufende Nut 34a umfassen (selbstverständlich können auch mehrere solcher spiralförmiger Nuten vorgesehen sein). Die Orientierung dieser Nut 34a ist entgegengesetzt zur Drehrichtung des Fräsers beim Abtragen von Gewebe. Wenn hier also der Fräser beim Rechtslauf Gewebe abträgt, so ist die Nut 34a in Linksdrehrichtung orientiert.

Dies hat zur Folge, dass beim Fräsen durch diese Nut 34a ein Kühlmittel, beispielsweise eine physiologische Salzlösung, zum distalen Ende des Fräsers geführt werden kann. Der Fräser kann daher im distalen Endbereich, wo er Kontakt zum Gewebe hat, gekühlt werden.

In Fig. 4 ist schliesslich ein Ausführungsbeispiel der Ausgestaltung der stirnseitigen Schneidezähne 2b dargestellt ("Bohrkrone"). Man erkennt hier, dass sich die Zähne 2b von der Innenwand beginnend in Richtung auf das distale Ende des Hohlfräsers hin nach aussen zuspitzen. Dadurch wird erreicht, dass dann, wenn der Hohlfräser einmal nicht ganz genau orthogonal auf das zu schneidende Gewebe aufgesetzt wird, dennoch nicht abgleiten kann, weil die zugespitzten Zähne 2b besser in das Gewebe einhaken. Um die Standzeit der Zähne 2b zu erhöhen, können die Zähne 2b an ihrer Spitze mit einem sehr kleinen Radius abgerundet sein.

Als Materialien für solche Fräser kommt rostfreier Stahl in Frage, der ggf. mit einer Titannitrid-Beschichtung (TiN-Beschichtung) versehen sein kann. Der Stahl kann auch wärmebehandelt sein, insbesondere gehärtet und/oder vergütet. Der Fräser ist sterilisierbar und kann demzufolge nach Reinigung und anschliessendem Sterilisieren wiederverwendet werden.

## Patentansprüche

1. Hohlfräser (1) zur Erstellung von im wesentlichen hohlzylindrischen Vertiefungen in menschlichem oder tierischem Gewebe, insbesondere zur Erstellung von Gewebesäulen, die an einer Erntestelle entnommen, zu einer Defektstelle transportiert und dort implantiert werden, wobei der Fräser Mittel (4,34a) zum Kühlen des nahe dem distalen Ende gelegenen Bereichs des Fräsers beim Abtragen von Gewebe aufweist,
**dadurch gekennzeichnet dass** der Fräser am distalen Ende stirnseitig angeordnete Zähne (2) zum Abtragen von Gewebe aufweist, und dadurch dass die Mittel zum Kühlen mindestens eine in der Aussenwand des Fräsers spiralförmig verlaufende, nach außen offene Nut (34a) umfassen, deren Orientierung entgegengesetzt zur Drehrichtung beim Abtragen von Gewebe ist, sodass beim Abtragen von Gewebe ein Kühlmittel, z.B. eine physiologische Salzlösung, durch die Nut (34a) zum distalen Ende des Fräsers gelangen kann.

2. Hohlfräser nach Anspruch 1, **dadurch gekennzeichnet, dass** die Mittel zum Kühlen diskrete Durchgangsbohrungen (4) umfassen, die nahe dem distalen Ende in der Wand des Fräsers ausgebildet sind.

3. Hohlfräser nach Anspruch 2, **dadurch gekennzeichnet, dass** in Umfangsrichtung betrachtet mehrere Durchgangsbohrungen (4) gleichmässig zueinander beabstandet in der Wand des Fräsers angeordnet sind, und dass in axialer Richtung betrachtet mehrere Lagen solcher in Umfangsrichtung gleichmässig zueinander beabstandeter Durchgangsbohrungen (4) in der Wand des Fräsers vorgesehen sind.

4. Hohlfräser nach einem der vorangehenden Ansprüche, **dadurch gekennzeichnet, dass** sich die stirnseitig angeordneten Schneidezähne (2b) von der Innenwand beginnend in Richtung auf das distale Ende des Hohlfräsers hin konisch nach aussen zuspitzen oder umgekehrt.

5. Hohlfräser nach einem der vorangehenden Ansprüche, **dadurch gekennzeichnet, dass** die Spitzen der stirnseitig angeordneten Schneidezähne (2b) abgerundet sind.

6. Hohlfräser nach einem der Ansprüche 1 bis 5, **dadurch gekennzeichnet, dass** im Bereich des distalen Endes des Fräsers der Innendurchmesser in proximaler Richtung betrachtet zunächst konstant ist und sich dann vergrössert.

7. Hohlfräser nach einem der Ansprüche 1 bis 5, **dadurch gekennzeichnet, dass** im Bereich des distalen Endes des Fräsers der Aussendurchmesser in proximaler Richtung betrachtet zunächst konstant ist und sich dann verringert.

8. Hohlfräser nach einem der vorangehenden Ansprüche, **dadurch gekennzeichnet, dass** im Bereich des distalen Endes der Fräsers Markierungen (31) zum Anzeigen der Eindringtiefe auf der Aussenwand vorgesehen sind.

9. Hohlfräser nach einem der vorangehenden Ansprüche, **dadurch gekennzeichnet, dass** auf der Aussenwand des Fräsers Markierungen (50,51) vorgesehen sind, welche sonstige Informationen über den Fräser enthalten.

## Claims

1. Hollow milling tool (1) for the production of substantially hollow cylindrical depressions in human or animal tissue, in particular for the production of tissue pillars which are removed at a harvest location, transported to a defect location and implanted there, wherein the milling tool has means (4, 34a) for the cooling of the region of the milling tool lying near the distal end during the removal of tissue,
**characterised in that** the milling tool has teeth (2) for the removal of tissue which are arranged at the distal end of the milling tool at the end face; and **in that** the means for the cooling comprise at least one groove (34a), which is outwardly open and extends in spiral shape in the outer wall of the milling tool, and the orientation of which is opposite to the direction of rotation during the removal of tissue so that when tissue is being removed a coolant, e.g. a physiological saline solution, can reach the distal end of the milling tool through the groove (34a).

2. Hollow milling tool in accordance with claim 1, **characterised in that** the means for the cooling comprise discrete passage bores (4) which are formed in the wall of the milling tool near the distal end.

3. Hollow milling tool in accordance with claim 2, **characterised in that**, when viewed in the peripheral direction, a plurality of passage bores (4) are arranged in the wall of the milling tool with a uniform mutual spacing; and **in that**, when viewed in the axial direction, a plurality of layers of such passage bores (4), which are mutually uniformly spaced when viewed in the peripheral direction, are provided in the wall of the milling tool.

4. Hollow milling tool in accordance with any one of the preceding claims, **characterised in that** the cutting teeth (2b), which are arranged at the end face, are conically outwardly tapered to a tip in the direction toward the distal end of the hollow milling tool, starting from the inner wall, or vice versa.

5. Hollow milling tool in accordance with any one of the preceding claims, **characterised in that** the tips of the cutting teeth (2b), which are arranged at the end face, are rounded off.

6. Hollow milling tool in accordance with any one of the claims 1 to 5, **characterised in that**, in the region of the distal end of the milling tool, the inner diameter is first constant and then enlarges, when viewed in the proximal direction.

7. Hollow milling tool in accordance with any one of the claims 1 to 5, **characterised in that**, in the region of the distal end of the milling tool, the outer diameter is first constant and then decreases, when viewed in the proximal direction.

8. Hollow milling tool in accordance with any one of the preceding claims, **characterised in that** markings (31) are provided on the outer wall in the region of the distal end of the milling tool for the indication of the penetration depth.

9. Hollow milling tool in accordance with any one of the preceding claims **characterised in that** markings (50, 51) are provided on the outer wall of the milling tool which contain other information on the milling tool.

## Revendications

1. Fraise creuse (1) destinée à la réalisation de renfoncements sensiblement cylindriques creux dans des tissus humains ou animaux, notamment à la réalisation de colonnes de tissu, qui sont prélevées au niveau d'un point de prélèvement, amenées à un emplacement altéré et implantées à ce niveau, la fraise comportant des moyens (4, 34a) pour le refroidissement de la zone de la fraise située à proximité de l'extrémité distale lors du prélèvement de tissu, **caractérisée en ce que** la fraise présente des dents (2) disposées sur la face frontale de l'extrémité distale pour le prélèvement de tissu, et **en ce que** les moyens de refroidissement comprennent au moins une rainure (34a) ouverte vers l'extérieur s'étendant en forme de spirale dans la paroi extérieure de la fraise, dont l'orientation est opposée au sens de rotation lors du prélèvement de tissu de sorte que, lors du prélèvement de tissu, un agent de refroidissement, par exemple une solution saline physiologique, puisse parvenir à travers la rainure (34a) vers l'extrémité distale de la fraise.

2. Fraise creuse selon la revendication 1, **caractérisée en ce que** les moyens de refroidissement comportent des perçages de passage (4) discrets, qui sont pratiqués dans la paroi de la fraise à proximité de l'extrémité distale.

3. Fraise creuse selon la revendication 2,
**caractérisée en ce que**, vu dans la direction périphérique, plusieurs perçages de passage (4) espacés régulièrement les uns des autres sont pratiqués dans la paroi de la fraise, et **en ce que**, vu dans la direction axiale, plusieurs couches de tels perçages de passage (4) espacés régulièrement les uns des autres dans la direction périphérique sont prévues dans la paroi de la fraise.

4. Fraise creuse selon l'une quelconque des revendications précédentes, **caractérisée en ce que**, à partir de la paroi intérieure, les dents de découpe (2b) disposées en face frontale sont effilées de façon conique vers l'extérieur en direction de l'extrémité distale de la fraise creuse, ou inversement.

5. Fraise creuse selon l'une quelconque des revendications précédentes, **caractérisée en ce que** les pointes des dents de découpe (2b) disposées en face frontale sont arrondies.

6. Fraise creuse selon l'une quelconque des revendications 1 à 5, **caractérisée en ce que**, dans la zone de l'extrémité distale de la fraise, le diamètre intérieur est d'abord constant vu dans la direction proximale, et augmente ensuite.

7. Fraise creuse selon l'une quelconque des revendications 1 à 5, **caractérisée en ce que**, dans la zone de l'extrémité distale de la fraise, le diamètre extérieur est d'abord constant vu dans la direction proximale, et se réduit ensuite.

8. Fraise creuse selon l'une quelconque des revendications précédentes, **caractérisée en ce que**, dans la zone de l'extrémité distale de la fraise, des marquages (31) destinés à l'indication de la profondeur de pénétration sont prévus sur la paroi extérieure.

9. Fraise creuse selon l'une quelconque des revendications précédentes, **caractérisée en ce que** des marquages (50, 51) contenant diverses informations relatives à la fraise sont prévus sur la paroi extérieure de la fraise.
